(19) Europäisches Patentamt
European Patent Office
Office européen des brevets

(11) Numéro de publication: **0 645 375 A1**

(12) **DEMANDE DE BREVET EUROPEEN**

(21) Numéro de dépôt: **94401733.4**

(22) Date de dépôt: **27.07.94**

(51) Int. Cl.[6]: **C07D 209/30**, A61K 31/40

(30) Priorité: **30.07.93 FR 9309452**

(43) Date de publication de la demande:
**29.03.95 Bulletin 95/13**

(84) Etats contractants désignés:
**AT BE CH DE DK ES FR GB GR IE IT LI LU NL PT SE**

(71) Demandeur: **ELF SANOFI**
**32-34, rue Marbeuf**
**F-75008 Paris (FR)**

(72) Inventeur: **Herbert, Jean-Marc**
**10, rue de l'Amandier**
**F-31170 Tournefeuille (FR)**
Inventeur: **Chatelain, Pierre**
**111, avenue du Haras**
**B-1150 Bruxelles (BE)**

(74) Mandataire: **Le Guen, Gérard et al**
**CABINET LAVOIX**
**2, place d'Estienne d'Orves**
**F-75441 Paris Cédex 09 (FR)**

(54) **Utilisation de dérivés de benzène-sulfonyl-indole pour la préparation de médicaments.**

(57) Utilisation d'au moins un dérivé d'indole de formule générale :

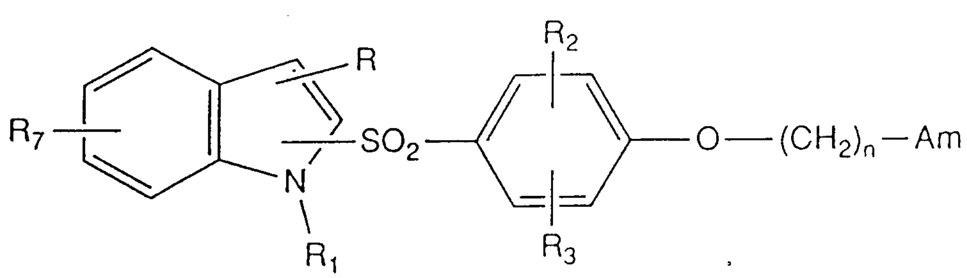

ou d'un de ses sels pharmaceutiquement acceptables, dans laquelle :
Am représente un groupement

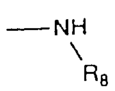

(a)

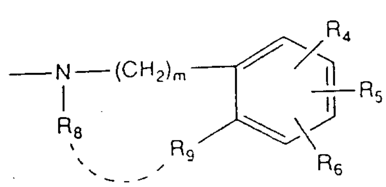

(b)

R représente un radical alkyle en $C_1$-$C_6$ ou cycloalkyle en $C_3$-$C_6$
$R_1$ représente l'hydrogène ou un radical alkyle en $C_1$-$C_4$
$R_2$ et $R_3$, identiques ou différents représentent l'hydrogène, le radical méthyle ou éthyle ou un halogène
$R_4$, $R_5$ et $R_6$, identiques ou différents, représentent l'hydrogène, un radical alkyle en $C_1$-$C_4$, alkoxy en $C_1$-$C_4$

EP 0 645 375 A1

ou un halogène

$R_7$ représente l'hydrogène ou un halogène

$R_8$ représente l'hydrogène ou un radical alkyle en $C_1$-$C_4$

$R_9$ représente l'hydrogène ou lorsque $R_8$ et $R_9$ sont pris ensemble, forment le radical méthylène

m représente 1 ou 2

n représente 2, 3 ou 4

pour la préparation à' un médicament destiné à prévenir ou à traiter des affections pathologiques impliquant une prolifération de cellules musculaires lisses.

La présente invention se rapporte, d'une manière générale, à une nouvelle utilisation de dérivés d'indole pour la préparation de médicaments.

En particulier, l'invention concerne une nouvelle utilisation de dérivés de benzènesulfonyl-indole de formule générale :

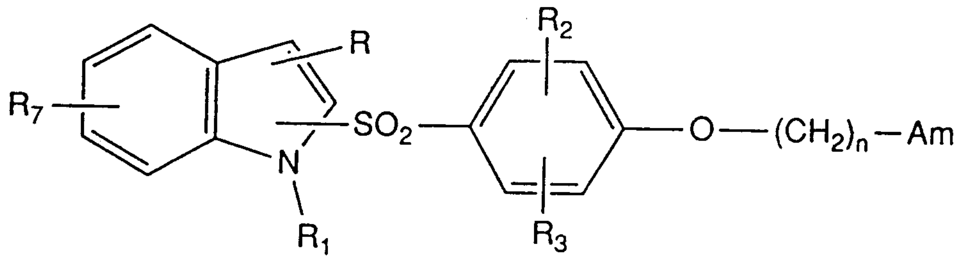

dans laquelle :

Am représente un groupement

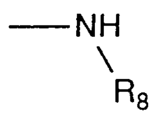

(a)

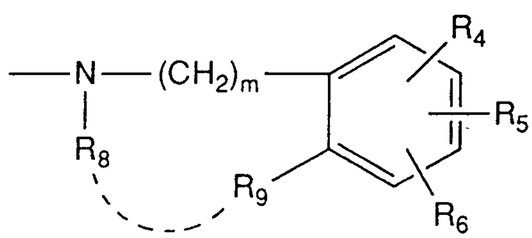

(b)

R représente un radical alkyle en $C_1$-$C_6$ ou cycloalkyle en $C_3$-$C_6$

$R_1$ représente l'hydrogène ou un radical alkyle en $C_1$-$C_4$

$R_2$ et $R_3$, identiques ou différents représentent l'hydrogène, le radical méthyle ou éthyle ou un halogène

$R_4$, $R_5$ et $R_6$, identiques ou différents, représentent l'hydrogène, un radical alkyle en $C_1$-$C_4$, alkoxy en $C_1$-$C_4$ ou un halogène

$R_7$ représente l'hydrogène ou un halogène

$R_8$ représente l'hydrogène ou un radical alkyle en $C_1$-$C_4$

$R_9$ représente l'hydrogène ou lorsque $R_8$ et $R_9$ sont pris ensemble, forment le radical méthylène

m représente 1 ou 2

n représente 2, 3 ou 4

ainsi que leurs sels pharmaceutiquement acceptables, pour la préparation de médicaments.

Ainsi, en tenant compte des valeurs ci-dessus :

R peut représenter notamment un radical méthyle, éthyle, n-propyle, isopropyle, n-butyle,n-pentyle, n-hexyle ou cyclopropyle,

$R_1$ peut représenter notamment le radical méthyle,

$R_2$ et $R_3$ peuvent représenter notamment le chlore ou le brome,

$R_4$, $R_5$ et $R_6$ peuvent représenter notamment le radical méthyle, méthoxy ou le chlore,

$R_7$ peut représenter notamment le chlore,

$R_8$ peut représenter notamment le radical méthyle.

Une classe particulière de composés de formule I est représentée par ceux dans lesquels R représente le radical isopropyle ou cyclopropyle et $R_2$ et $R_3$, identiques, représentent l'hydrogène.

Une autre classe de composés de formule I est représentée par ces composés dans lesquels $R_7$ représente l'hydrogène d'une part et l'un des $R_4$, $R_5$ et $R_6$ représente l'hydrogène, le radical méthyle ou méthoxy les deux autres représentant le radical méthyle ou méthoxy d'autre part.

Une autre classe de composés de formule I est formée par ces composés dans lesquels la chaine alkoxybenzènesulfonyle substituée se trouve en position 2 du cycle indole et en particulier représente le groupement 4-{3-[N-méthyl-N-(3,4-diméthoxyphénéthyl) amino]propoxy}benznèsulfonyle.

Comme exemples de sels pharmaceutiquement acceptables des composés de formule I on peut citer des sels formés à partir d'un acide organique tels que les oxalate, maléate, fumarate, méthanesulfonate, benzoate, ascorbate, pamoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate et théophylline acétate ainsi que des sels formés à partir d'un acide aminé tel que le sel de lysine ou de l'histidine ou encore des sels formés à partir d'un acide inorganique tels que les chlorhydrate, bromhydrate, sulfate, sulfamate, phosphate et nitrate.

Durant les deux dernières décennies, on a porté un intérêt tout particulier au rôle joué par la prolifération des cellules musculaires lisses de l'intima dans le développement de l'athérosclérose.

La caractérisation de lésions expérimentales induites chez l'animal et l'étude histologique de lésions survenant au niveau d'artères coronaires humaines ont démontré qu'une plaque athéroscléreuse débute comme une prolifération nodulaire de cellules musculaires lisses qui se complique, par la suite, par l' accumulation de cellules spumeuses chargées en lipides, de cellules nécrosées et de calcification.

Ces découvertes suggèrent que des agents interférant avec la prolifération de cellules musculaires lisses peuvent présenter un intérêt thérapeutique dans la prévention et le traitement de l'athérosclérose.

Les antagonistes du calcium constituent un groupe d'agents pharmacologiquement actifs qui inhibent les processus dépendant de l'ion $Ca^{++}$. Leur principal mécanisme d'action s'établit par blocage de l'entrée des ions calcium dans les cellules. En clinique, les antagonistes du calcium se sont révélés efficaces dans le traitement de l'angor, de l'hypertension et des arythmies cardiaques.

On a largement rapporté dans la littérature scientifique que des agents anticalciques inhibent sélectivement la prolifération de cellules musculaires lisses survenant après une lésion expérimentale. Ces antagonistes du calcium, par ce mécanisme, apporteront, en conséquence, un effet bénéfique dans la prévention et le traitement de l'athérosclérose.

Parmi les agents antagonistes du $Ca^{++}$ qui ont été étudiés comme inhibiteurs de la prolifération des cellules musculaires lisses, on peut citer la nifédipine, le diltiazem ou le vérapamil. Ce dernier se caractérise par la présence d'une chaîne N-méthyl-N-(3,4-diméthoxy-phénéthyl) amino dans sa molécule, de même que d'autres agents anticalciques décrits dans les demandes de brevets EP - A - 0302793, 0382618, 0382628 et 0382629, notamment la 3-isopropyl-1-[4-{3-[N-méthyl-N-(3,4-diméthoxy-phénéthyl)-amino] propoxy} benzènesulfonyl]-indolizine ou fantofarone.

Or, on a maintenant trouvé de manière surprenante que des dérivés de l'indole comportant une telle chaîne N-méthyl-N-(3,4-diméthoxy-phénéthyl) amino et plus généralement, les dérivés d'indole de formule I, objets des demandes de brevets cités précédemment, ainsi que leurs sels pharmaceutiquement acceptables, sont doués de remarquables propriétés biochimiques et pharmacologiques vis-à-vis de la paroi vasculaire. En effet, ces composés se sont révélés capables d'inhiber la prolifération des cellules musculaires lisses tels que par exemple des cellules artérielles aortiques, mésentériques, fémorales ou carotidiennes.

Par contre, le vérapamil et la fantofarone, dans les conditions expérimentales étudiées, se sont généralement révélés dépourvus de telles propriétés ou se sont montrés doués de ces propriétés à des doses élevées voire infra-toxiques.

Les composés de formule I ainsi que leurs sels pharmaceutiquement acceptables de même que les compositions pharmaceutiques ou vétérinaires les contenant seront, par conséquent, utiles en vue de prévenir ou de traiter par exemple la resténose après angioplastie transluminale coronarienne ou l'athérosclérose dans laquelle la prolifération des cellules musculaires lisses vasculaires a été décrite comme un événement important de la pathogénèse.

En conséquence, un premier objet de l'invention se rapporte à l'utilisation d'au moins un dérivé d'indole de formule I ou d'un de ses sels pharmaceutiquement acceptables, pour la préparation d' un médicament destiné à prévenir ou à traiter des affections pathologiques impliquant une prolifération des cellules musculaires lisses.

A titre d'exemple de composés utiles dans cette nouvelle utilisation, on peut citer les dérivés d'indole de formule I ci-dessous qui ont été décrits dans les brevets précités :

Oxalate acide de 1-méthyl-3-isopropyl-2-[4-{3-(N-méthyl-N-(3,4-diméthoxy-phénéthyl) amino]-propoxy}benzènesulfonyl]-indole (SR 33805 A).

Fumarate acide de 1-méthyl-2-{4-[3-(6,7-diméthoxy-1,2,3,4-tetrahydro-N-isoquinolin-2-yl)propoxy]-benzènesulfonyl})-3-isopropylindole (SR 33905 A).

Chlorhydrate de 1-méthyl-3-isopropyl-2-[4-{3-[N-méthyl-N-(3,4,5-triméthoxy-phénéthyl)amino]propoxy} benzènesulfonyl]-indole (SR 34041 A)
Chlorhydrate de 1-méthyl-3-isopropyl-2-[4-{3-[N-(3,4,5-triméthyl-phénéthyl) amino]propoxy} benzènesulfonyl] -indole (SR 34079 A)

Parmi les composés de formule I, le 1-méthyl-3-isopropyl-2-[4-{3-[N-méthyl-N-(3,4-diméthoxy-phénéthyl)amino]propoxy}benzènesulfonyl]-indole, ci-après dénommé SR 33805, de formule :

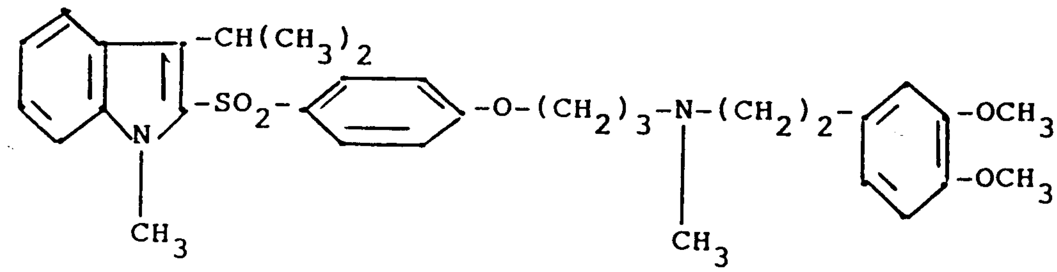

ainsi que ses sels pharmaceutiquement acceptables, a montré les potentialités inhibitrices les plus intéressantes vis-à-vis de la prolifération des cellules musculaires lisses. A ce titre, le SR 33805 et ses sels pharmaceutiquement acceptables constituent des conposés préférés selon l'invention.

Le SR 33805 sous forme de son oxalate acide a été décrit spécifiquement dans la demande de brevet EP - A - 0302793 alors qu'aucun autre sel de ce composé n'y a été ni cité ni exemplifié.

Toutefois, il est reconnu que l'anion oxalate ne constitue pas le contre-ion de premier choix pour la formation de sels pharmaceutiquement acceptables.

Or, on a trouvé que le méthanesulfonate du SR 33805, ci-après dénommé SR 33805 C, constitue un composé particulièrement intéressant en raison d'un ensemble de critères qui lui confèrent des qualités essentielles pour une utilisation dans la préparation d'un médicament, plus généralement une composition pharmaceutique ou vétérinaire.

Par exemple, au contraire d'autres sels du SR 33805, le méthanesulfonate de ce composé, étant donné sa bonne solubilité aqueuse, s'est révélé beaucoup plus approprié pour la réalisation de solutions injectables par exemple des solutions injectables à 5 % ou même de concentrations inférieures.

Des tests de solubilité effectués avec le SR 33805 C, le SR 33805 A (oxalate acide du SR 33805) et le SR 33805 B (fumarate du SR 33805) ont révélé les taux de solubilité ci-dessous :

| Résultats | (1) | (2) | (3) |
|---|---|---|---|
| SR 33805 A | 0,007% | 0,019% | 0,031% |
| SR 33805 B | 0,015% | 0,010% | 0,026% |
| SR 33805 C | 6,75% | 7,99% | 9,83% |

(1) solubilité dans l'eau à 25 °C après agitation pendant 72 heures
(2) solubilité dans l'eau de pH = 3,6
(3) solubilité après ajout du produit à tester à de l'eau à 70 °C, agitation et maintien de l'agitation durant 72 h à 25 °C.

De même, le SR 33805 C ne montre pas de signes d'hygroscopicité, possède une bonne stabilité dans différentes conditions de conservation et peut être obtenu sous une forme cristalline compatible avec une utilisation pour la préparation de formes pharmaceutiques ou vétérinaires contrairement à d'autres sels du SR 33805 qui se sont révélés très inférieurs dans ces critères.

En conséquence, un autre objet de l'invention se rapporte au méthanesulfonate du SR 33805 en tant que nouveau produit.

De même, l'invention concerne un médicament essentiellement une composition pharmaceutique ou vétérinaire contenant comme principe actif le méthanesulfonate du SR 33805 en association avec un véhicule pharmaceutique ou un excipient approprié.

Pour les raisons exposées précédemment le méthanesulfonate du SR 33805 constitue un composé particulièrement préféré selon l'invention.

En outre, un autre dérivé d'indole de formule I s'est révélé lui aussi d'un intérêt particulier en raison de son activité inhibitrice importante vis-à-vis de la prolifération des cellules musculaires lisses et de son action très modérée voire nulle sur le système cardiovasculaire à savoir le 1-méthyl-3-isopropyl-2-{4-[3-(N-méthylamino)propoxy]benzènesufonyl}indole ci-après dénommé SR 34549.

Ce composé, en effet, s'est révélé dépourvu, in vitro, de propriétés inhibitrices de la translocation calcique.

En conséquence, l'invention se rapporte également au SR 34549 et plus généralement, aux nouveaux dérivés d'indole de formule I dans laquelle Am représente un groupement $-NHR_8$,
ainsi qu'à leurs sels pharmaceutiquement acceptables.

Les propriétés inhibitrices des composés de formule I ainsi que de leurs sels pharmaceutiquement acceptables vis-à-vis de la prolifération des cellules musculaires lisses ont été mises en évidence au moyen de différents tests biochimiques et pharmacologiques résumés ci-dessous.

I. **Tests "in vitro"**

On a effectué, "in vitro" des tests à partir de cellules musculaires lisses aortiques humaines au repos ($10^3$ cellules par test).

On a incubé ces cellules en présence de milieu eagle minimum additionné de 5% d'un agent inducteur d'une prolifération cellulaire. Ces tests ont été pratiqués avec différentes doses croissantes du composé à étudier comparativement à des témoins.

Après 3 jours d'incubation, on a arrêté la croissance des cellules par ajout de trypsine et on a exprimé les résultats en concentrations permettant d'inhiber de 50% la prolifération cellulaire ($CI_{50}$).

On a enregistré les résultats suivants comparativement à la fantofarone, au vérapamil ou la nifédipine.

a) Agent inducteur : sérum foetal de veau

| Composé étudié | $CI_{50}$ (µM) |
|---|---|
| SR 33805 C | 0,9 |
| Fantofarone | 5,5 |
| Vérapamil | 10,- |

Des tests analogues pratiqués avec d'autres composés de formule I sous forme de sels pharmaceutiquement acceptables ont révélé des $CI_{50}$ comprises entre 0,09 et 3 µM.

b) Agent inducteur : facteur de croissance foetal

| Composé étudié | $CI_{50}$ (µM) |
|---|---|
| SR 33805 C | 0,6 |
| Fantofarone | 2,- |
| Nifédipine | 1,- |

c) Agent inducteur : facteur de croissance issu de plaquettes

| Composé étudié | $CI_{50}$ (µM) |
|---|---|
| SR 33805 C | 0,3 |
| Fantofarone | > 10,- |
| Nifédipine | > 10,- |

II. **Tests "in vivo"**

**1) *Inhibition de la surcharge en $Ca^{++}$ après traitement à la vitamine $D_3$ chez le rat***

On a pratiqué un test sur rats auxquels on a administré 300.000 UI/kg de vitamine $D_3$ par voie intramusculaire puis, par voie orale, une dose journalière du composé à étudier. On a poursuivi pendant 3 jours le traitement par voie orale au moyen du composé à tester, on a sacrifié les animaux puis on a dosé le $Ca^{++}$ dans différents territoires artériels (fémoral, mésentérique, aortique...). Dans ce test, le SR

33805 C exerce, dès la dose de 2 mg/kg administrée deux fois par jour, un effet significatif d'inhibition de la surcharge en $Ca^{++}$ au niveau de l'aorte. Un effet significatif analogue a été enregistré au niveau de l'artère mésentérique dès la dose de 5 mg/kg administrée 2 fois par jour.

A titre de comparaison, la fantofarone testée dans les mêmes conditions expérimentales a provoqué une diminution significative du contenu total en $Ca^{++}$ à partir de 100 mg/kg administrés 2 fois par jour.

Le SR 33805 C s'est donc révélé dans ce test de 20 à 50 fois plus actif que la fantofarone.

Dans les mêmes conditions expérimentales le vérapamil, quant à lui, s'est montré de 15 à 30 fois moins actif que le SR 33805 C.

2) Inhibition de la prolifération myointimale induite au niveau des artères carotides chez le lapin après lésion de l'endothélium

On a utilisé, à cet effet, des lapins dont on a oté l'endothélium de l'artère carotide.

Deux heures avant la lésion, on a administré le composé à étudier par voie orale. On a poursuivi le traitement par voie orale, journellement pendant 16 jours puis on a déterminé les surfaces de l'intima et de la média par mesure morphométrique.

Les résultats ont montré qu'après 16 jours de traitement dès la dose de 1,5 mg/kg, le SR 33805 C inhibe de façon significative (-58%) la prolifération myointimale.

Aux doses maximales testées, la nifédipine (6 mg/kg ou 2 fois 6 mg/kg), la fantofarone (12 mg/kg), le vérapamil (2 fois 6 mg/kg) ou le diltiazem (2 fois 6 mg/kg) n'ont pas présenté d'effet significatif sur l'hyperplasie myointimale.

Ces différents résultats montrent que les dérivés d'indole de formule I et leurs sels pharmaceutiquement acceptables, spécialement le SR 33805 sous forme de son méthanesulfonate, se révèlent de loin supérieurs à d'autres agents anticalciques à savoir le vérapamil, la fantofarone ou la nifédipine, comme inhibiteurs de la prolifération des cellules musculaires lisses.

Les dérivés d'indole de formule I ainsi que leurs sels pharmaceutiquement acceptables peuvent être préparés comme décrit dans les demandes de brevets citées précédemment.

Par exemple, on peut préparer ces composés en traitant un dérivé hydroxy-4 benzènesulfonyle de formule générale :

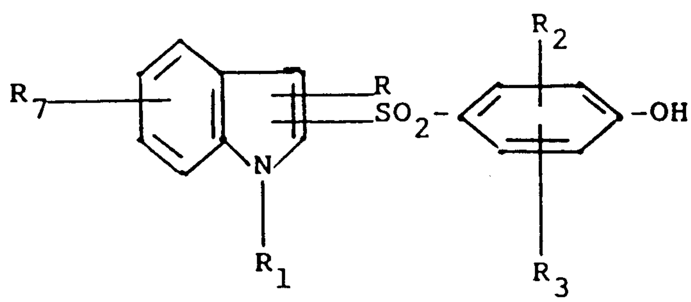

II

dans laquelle R, $R_1$, $R_2$, $R_3$ et $R_7$ ont la même signification que précédemment, avec un halogénure de formule générale :

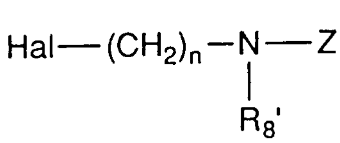

or

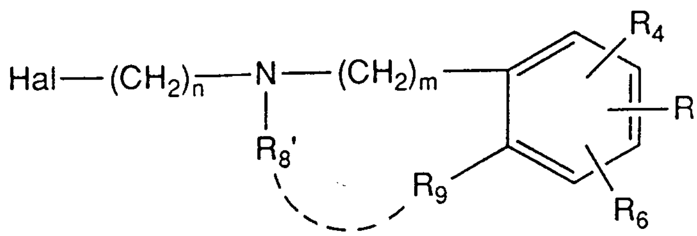

III

III'

dans laquelle $R_4$,$R_5$,$R_6$,$R_8$,$R_9$, m et n ont la même signification que précédemment, Hal représente un halogène, de préférence le brome, $R'_8$ représente un groupement alkyle en $C_1$-$C_4$ et Z représente un groupement protecteur tel que par exemple un groupement terbutoxycarbonyle (BOC), la réaction étant conduite en présence d'un agent basique tel qu'un carbonate de métal alcalin, un hydroxyde de métal alcalin, un hydrure de métal alcalin ou un alcoolate de métal alcalin pour obtenir :

- soit des composés de formule dans laquelle Am représente un groupement (b)
- soit des composés N-protégés que l'on traite par l'acide trifluoroacétique pour obtenir des composés de formule I dans laquelle Am représente un groupement (a),

composés de formule I que l'on peut si on le désire, faire réagir avec un acide approprié pour former un sel pharmaceutiquement acceptable.

En variante, on peut également préparer les composés de formule I en faisant réagir un dérivé d'indole de formule II avec un dihalogénoalkane de formule générale :

$Hal-(CH_2)_n-Hal$ IV

dans laquelle Hal et n ont la même signification que précédemment, la réaction ayant lieu dans un solvant tel que la méthyl éthyl cétone on le N,N-diméthylformamide en présence d'un agent basique tel qu'un carbonate de métal alcalin, un hydrure de métal alcalin, un hydroxyde de métal alcalin ou un alcoolate de métal alcalin pour obtenir un dérivé haloalkoxy benzènesulfonyl-indole.

On fait alors réagir ce dérivé haloalkoxy benzènesulfonyl-indole avec une amine de formule générale :

H-Am V

dans laquelle Am a la même signification que précédemment, la réaction ayant lieu dans un solvant approprié tel qu'un solvant polaire ou non polaire et en présence d'un agent accepteur d'acide ou dans un excès d'amine de formule V, pour obtenir les composés de formule I que l'on peut, si on le désire, faire réagir avec un acide approprié, pour former un sel pharmaceutiquement acceptable de ce composé.

Le médicament selon l'invention, lorsqu'il est constitué d'une composition pharmaceutique ou vétérinaire, peut, lui aussi, être préparé de manière connue tel que décrit dans les demandes de brevets énumérées ci-dessus, essentiellement par association d'au moins un dérivé d'indole de formule I ou d'un de ses sels pharmaceutiquement acceptables, avec un véhicule pharmaceutique ou un excipient approprié

Enfin, les dérivés d'indole de formule I et leurs sels pharmaceutiquement acceptables peuvent être utilisés à des fins thérapeutiques selon des posologies analogues à celles préconisées dans les demandes de brevets citées ci-dessus généralement pour un être humain de 60 kgs à des doses journalières allant de 2 à 800 mg selon la voie d'administration choisie.

Les Exemples non limitatifs suivants illustrent la préparation du SR 33805 C ainsi que d'une composition pharmaceutique le contenant :

## EXEMPLE 1

Préparation du méthanesulfonate de 1-méthyl-3-isopropyl-2-[4-{3-[N-méthyl-N-(3,4-diméthoxy-phénethyl)-aminolpropoxy} benzènesulfonyl]-indole

A un mélange de 9,9 g (0,003 mole) de 2-(4-hydroxy-benzènesulfonyl)-3-isopropyl-1-méthyl-indole dans 165 ml de N,N-diméthylformamide, on ajoute 29 g (0,21 mole) de carbonate de potassium anhydre et finement broyé. On agite durant 0,5 heure et on ajoute 13,3 g (0,033 mole) d'oxalate acide de 1-chloro-3-[N-méthyl-N-(3,4-diméthoxy-phénéthyl)amino]-propyle à 90%.

On chauffe à 100 °C pendant 1 heure et on laisse revenir à température ambiante sous agitation. On verse dans 450 ml d'eau et de glace et on agite durant 0,25 heure. On extrait avec 3 fois 150 ml d'éther diéthylique, lave deux fois avec 150 ml d'eau, et on sèche sur sulfate de sodium.

A la solution éthérée obtenue, on ajoute 3,5 g d'acide méthanesulfonique dissous dans 50 ml d'éther éthylique et on laisse cristalliser pendant 24 heures. On essore, lave à l'éther éthylique et sèche sous vide à 50 °C.

De cette manière, on obtient le méthanesulfonate de 1-méthyl-3-isopropyl-2-[4-{3-[N-méthyl-N-(3,4-diméthoxy-phénéthyl)amino] propoxy} benzènesulfonyl]-indole sous forme d'un solide blanc cristallin.
P.F. : 166 °C (6/4 acétate d'éthyle/isopropanol).

**EXEMPLE 2**

Préparation du chlorydrate de 1-méthyl-3-isopropyl-2-{4-[3-(N-méthylamino)-propoxy]benzènesulfonyl}-indole:

On dissout 5,5 g (0,0145 mole) de 1-méthyl-3-isopropyl-2-[4-(3-bromopropoxy)benzènesufonyl]-indole dans 70 ml de N,N-diméthylformamide puis on refroidit la solution à 0°C. On ajoute alors 6,7 ml d'une solution de méthylamine à 3 % dans le méthanol et on laisse revenir à température ambiante durant 12 à 14 heures. On verse le mélange dans l'eau, on extrait avec de l'éther diéthylique puis on forme le chlorhydrate par ajout d'acide chlorhydrique dans l'éther diéthylique. On recristallise alors dans l'acétate d'éthyle.

De cette manière, on obtient 2,8 g de chlorhydrate de 1-méthyl-3-isopropyl-2-{4-[3-(N-méthylamino)-propoxy]benzènesulfonyl}-indole.

Rendement : 44,2 %

P.F. : 127°C(acétate d'éthyle)

**EXEMPLE 3**

Préparation de l'oxalate de 3-isopropyl-2-{4-[3-(N-méthylamino)propoxy]benzènesulfonyl}-indole :

On dissout 3,15 g (0,01 mole) de 3-isopropyl-2-(4-hydroxy-benzènesulfonyl)-indole dans 20 ml de diméthylsulfoxyde et on ajoute 2,5 g de carbonate de sodium broyé. On agite puis on ajoute 3,1 g (0,015 mole) de chlorure de N-BOC-N-méthyl-3-aminopropyle. On maintient le milieu à température ambiante durant 12 à 14 heures puis on chauffe durant 5 heures à 65-70°C.

On verse le mélange dans l'eau, extrait avec de l'acétate d'éthyle et lave la solution organique deux fois avec de l'eau. On sèche sur sulfate de sodium anhydre, titre et chasse le solvant, ce qui fournit 5,8 g d'un résidu huileux. On reprend le résidu dans 50 ml de dichlorométhane et 20 ml d'acide trifluoroacétique et on agite durant 1 heure à température ambiante. On évapore alors à sec (température du bain : 50°C) et on reprend le résidu dans de l'eau. On ajoute une solution d'hydroxyde de sodium, extrait avec de l'éther diéthylique et lave avec de l'eau. On sèche et évapore le solvant, ce qui fournit 1,26 g d'un résidu que l'on purifie par chromatographie sur colonne de silice en utilisant le méthanol comme éluant. On obtient ainsi 0,560 g de produit désiré sous forme basique que l'on transforme en oxalate par ajout d'acide oxalique dans l'éther diéthylique.

De cette manière, on préparel'oxalate de 3-isopropyl-2-{4-[3-(N-méthylamino)propoxy]-benzènesulfonyl}-indole.

Rendement : 14,5 %

P.F. : 115°c(isopropanol)

En utilisant les méthodes décrites précédemment, on a également préparé le chlorhydrate de 3-isopropyl-2-{4-(3-[N-(3,4-diméthoxyphénéthyl)amino]propoxy)benzènesulfonyl}-indole (**exemple 4**).

P.F. : 205°C (acétate d'éthyle).

**EXEMPLE 5**

Selon des techniques pharmaceutiques connues, on a préparé des gélules de composition suivante :

| Ingrédients | Mg |
|---|---|
| SR 33805 C | 50 |
| Lactose | 25 |
| Stéarate de magnésium | 25 |
| | 100 |

## Revendications

**1.** Utilisation d'au moins un dérivé d'indole de formule générale :

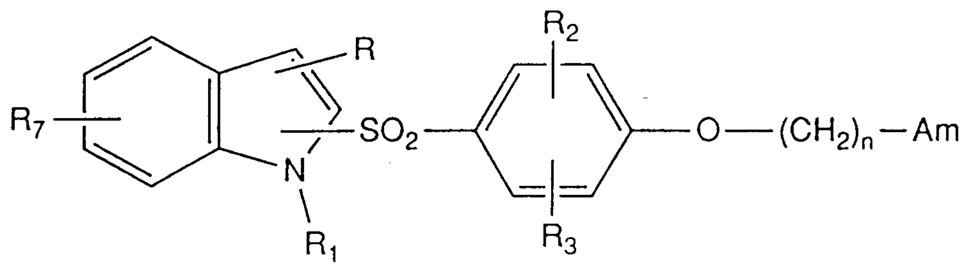

ou d'un de ses sels pharmaceutiquement acceptables, dans laquelle :

Am représente un groupement

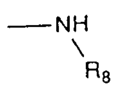

(a)

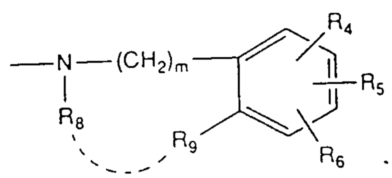

(b)

R représente un radical alkyle en $C_1$-$C_6$ ou cycloalkyle en $C_3$-$C_6$

$R_1$ représente l'hydrogène ou un radical alkyle en $C_1$-$C_4$

$R_2$ et $R_3$, identiques ou différents représentent l'hydrogène, le radical méthyle ou éthyle ou un halogène

$R_4$, $R_5$ et $R_6$, identiques ou différents, représentent l'hydrogène, un radical alkyle en $C_1$-$C_4$, alkoxy en $C_1$-$C_4$ ou un halogène

$R_7$ représente l'hydrogène ou un halogène

$R_8$ représente l'hydrogène ou un radical alkyle en $C_1$-$C_4$

$R_9$ représente l'hydrogène ou lorsque $R_8$ et $R_9$ sont pris ensemble, forment le radical méthylène

m représente 1 ou 2

n représente 2, 3 ou 4

pour la préparation d'un médicament destiné à prévenir ou à traiter des affections pathologiques impliquant une prolifération de cellules musculaires lisses.

**2.** Utilisation selon la Revendication 1 dans laquelle :

R représente un radical méthyle, éthyle, n-propyle, isopropyle, n-butyle, n-pentyle, n-hexyle ou cyclopropyle,

$R_1$ représente le radical méthyle,

$R_2$ et $R_3$ représentent le chlore ou le brome

$R_4$, $R_5$, $R_6$ représentent le radical méthyle ou méthoxy ou le chlore,

$R_7$ représente le chlore,

$R_8$ représente le radical méthyle.

**3.** Utilisation selon la Revendication 1 dans laquelle R représente le radical isopropyle ou cyclopropyle et $R_2$ et $R_3$, identiques représentent l'hydrogène.

**4.** Utilisation selon la Revendication 1 dans laquelle $R_7$ représente l'hydrogène et l'un des $R_4$, $R_5$ et $R_6$ représente l'hydrogène ou le radical méthyle ou méthoxy, les deux autres représentant le radical méthyle ou méthoxy.

5. Utilisation selon la Revendication 1 d'au moins un dérivé d'indole de formule générale :

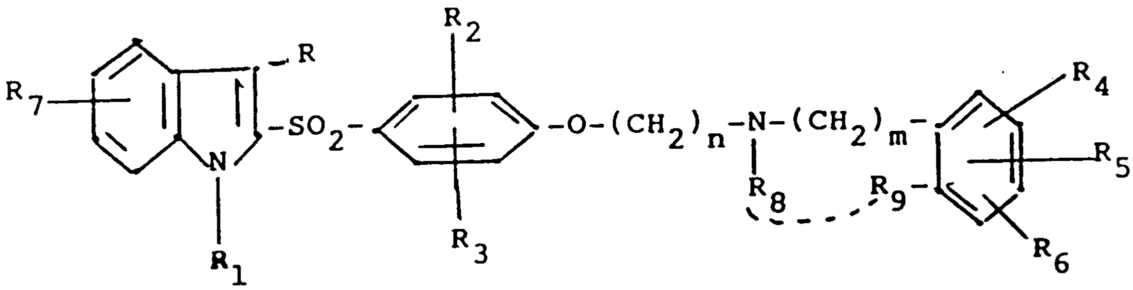

ou d'un de ses sels pharmaceutiquement acceptables, dans laquelle R, $R_1$, $R_2$, $R_3$, $R_4$, $R_5$, $R_6$, $R_7$, $R_8$, $R_9$, n et m ont la même signification que dans la Revendication 1.

6. Utilisation selon la Revendication 5 d'au moins un dérivé d'indole de formule générale :

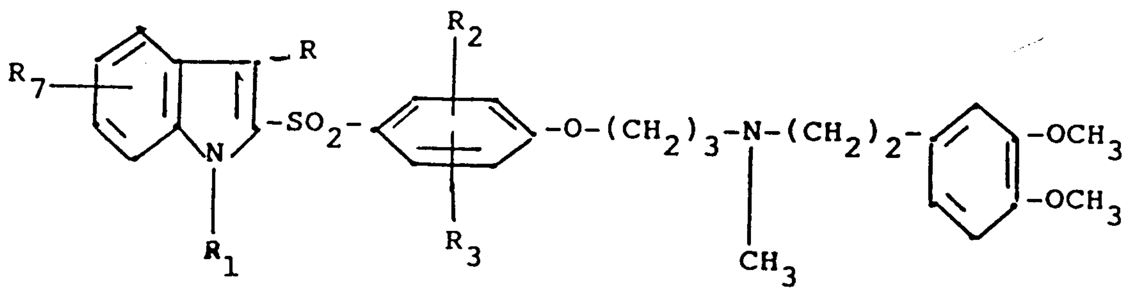

ou d'un de ses sels pharmaceutiquement acceptables, dans laquelle R, $R_1$, $R_2$, $R_3$ et $R_7$ ont la même signification que précédemment.

7. Utilisation selon la Revendication 1 ou 6 du 1-méthyl-3-isopropyl-2-[4-{3-[N-méthyl-N-(3,4-diméthoxy-phénéthyl)amino]propoxy} benzènesulfonyl]-indole ou d'un de ses sels pharmaceutiquement acceptables.

8. Utilisation selon la Revendication 7 du méthanesulfonate de 1-méthyl-3-isopropyl-2-[4-{3-[N-méthyl-N-(3,4-diméthoxy-phénéthyl)amino]propoxy} benzènesulfonyl]-indole.

9. Utilisation selon une des Revendications 1 à 8 pour la préparation d'un médicament destiné à prévenir ou à traiter la restenose après angioplastie transluminale coronarienne.

10. Utilisation selon une des Revendications 1 à 8 pour la préparation d'un médicament destiné à prévenir ou à traiter l'athérosclérose.

11. Méthanesulfonate du 1-méthyl-3-isopropyl-2-[4-{3-[N-méthyl-N-(3,4-diméthoxy-phénéthyl)amino]-propoxy} benzènesulfonyl]-indole.

12. Composition pharmaceutique ou vétérinaire caractérisée en ce qu'elle contient comme principe actif le méthanesulfonate de 1-méthyl-3-isopropyl-2-[4-{3-[N-méthyl-N-(3,4-diméthoxy-phénéthyl)amino]-propoxy} benzènesulfonyl]-indole, en association avec un véhicule pharmaceutique ou un excipient approprié.

13. Utilisation selon la revendication 1, dans laquelle Am représente - $NHR_8$

14. Utilisation selon la revendication 1 du 1-méthyl-3-isopropyl-2-{4-[3-(N-méthylamino)propoxy-[benzènesulfonyl}indole ou d'un de ses sels pharmaceutiquement acceptable.

**15.** Dérivé d'indole de formule générale

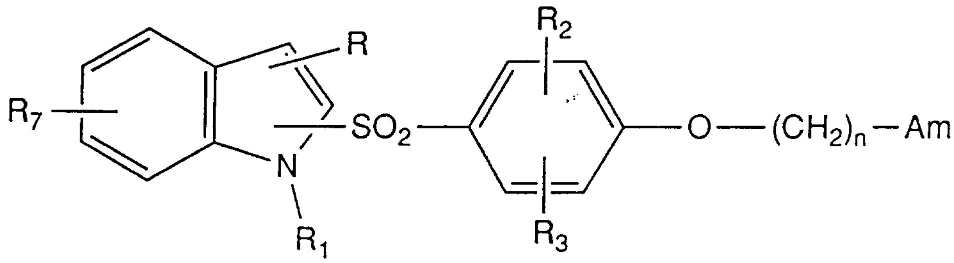

dans laquelle
Am représente $-NHR_8$
R représente un radical alkyle en $C_1$-$C_6$ ou cycloalkyle en $C_3$-$C_6$
$R_1$ représente l'hydrogène ou un radical alkyle en $C_1$-$C_4$
$R_2$ et $R_3$, identiques ou différents représentent l'hydrogène, le radical méthyle ou éthyle ou un halogène
$R_4$, $R_5$ et $R_6$, identiques ou différents, représentent l'hydrogène, un radical alkyle en $C_1$-$C_4$, alkoxy en $C_1$-$C_4$ ou un halogène
$R_7$ représente l'hydrogène ou un halogène
$R_8$ représente l'hydrogène ou un radical alkyle en $C_1$-$C_4$
$R_9$ représente l'hydrogène ou lorsque $R_8$ et $R_9$ sont pris ensemble, forment le radical méthylène
m représente 1 ou 2
n représente 2, 3 ou 4
et ses sels pharmaceutiquement acceptables.

**16.** 1-méthyl-3-ispropyl-2-{4-[3-(N-méthylamino)propoxy]benzènesulfonyl}-indole, et ses sels pharmaceutiquement acceptables.

Office européen des brevets

# RAPPORT DE RECHERCHE EUROPEENNE

Numero de la demande
EP 94 40 1733

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X,D | EP-A-0 302 793 (SANOFI)<br>* page 21 - page 22; revendications 1,8,24; exemple 45 *<br>* page 4, ligne 27 * | 1-12 | C07D209/30<br>A61K31/40 |
| X,D | EP-A-0 382 629 (S.A.SANOFI-LABAZ N.V.)<br>* page 21 - page 24; revendications * | 1-12 | |
| X,D | EP-A-0 382 628 (S.A.SANOFI-LABAZ N.V.) | 1-12 | |
| X,D | EP-A-0 382 618 (SANOFI)<br>* page 30, ligne 21 - ligne 22; revendications *<br>* page 22, ligne 52 - page 24, ligne 20 * | 1-12 | |

DOMAINES TECHNIQUES RECHERCHES (Int.Cl.6)

C07D
A61K

Le présent rapport a été établi pour toutes les revendications

| Lieu de la recherche | Date d'achèvement de la recherche | Examinateur |
|---|---|---|
| LA HAYE | 31 Août 1994 | Bosma, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)